# EUROPEAN PATENT APPLICATION

(11) **EP 4 342 415 A2**
(43) Date of publication of application: **27.03.2024**
(21) Application number: 23198120.0
(22) Date of filing: 19.09.2023
(51) Int. Cl.: A61C 1/08, A61B 34/20

(54) **METHOD AND SYSTEM FOR GUIDING OF DENTAL IMPLANTATION**

(30) Priority: 20.09.2022 KR 20220118404; 20.10.2022 KR 20220135381
(71) Applicant: Dentium Co., Ltd., Gyeonggi-do 16229 (KR)
(72) Inventor: Lee, In Jae, 16229 Suwon-si,Gyeonggi-do, (KR); Sung, Mun Hyun, 16229 Suwon-si,Gyeonggi-do, (KR); Choi, Young Hee, 16507 Suwon-si, Gyeonggi-do, (KR); Heo, Jun Mu, 17056 Yongin-si,Gyeonggi-do (KR); Kim, Min Ji, 16509 Suwon-si, Gyeonggi-do (KR); Jung, Won Jae, 16705 Suwon-si,Gyeonggi-do (KR)
(74) Representative: von Bülow & Tamada

(57) **Abstract**

A method for guiding of dental implant according to the present invention includes: a first step of visually displaying a CT image on a display so that a position and direction of implant placement according to a procedure plan are identified; a second step of detecting an electromagnetic field from a base by a sensor installed on a handpiece; and a third step of calculating the position and direction of the handpiece with respect to a procedure target site through the electromagnetic field detected by the sensor and visually displaying the position and direction so that an operator checks the position and direction in real time through the display, in which a target to be visually displayed in the third step includes a three-dimensional virtual image of the handpiece that reflects the misaligned position or direction of the handpiece when the position or direction of the handpiece is different from a reference according to the procedure plan.

## Description

### BACKGROUND

### 1. FIELD

The present invention relates to a method and system for guiding of dental implant.

### 2. DESCRIPTION OF RELATED ART

As we enter an aging society in earnest, interest in medical technologies that can solve maintenance, treatment, or the like of health is increasing. In addition to orthopedic surgery closely related to bone health which is gaining prominence as we enter an aging age, dental implant procedures are also increasing.

The dental implants are artificial teeth that are placed into a gum bone and are used when there are no teeth or the surrounding teeth are weak. Implant treatment begins with evaluating the quantity and quality of bone at a placement site, determining a placement position and depth, and making a surgical plan. In the actual procedure, it is important to accurately form (drill) a hole in a jawbone at the planned position and depth. For the drilling, an operator uses a handpiece that allows the procedure to be performed by attaching and detaching an appropriate tool depending on the stage. In this case, even if the starting position is selected correctly, a direction may be misaligned during the drilling process or the depth may not be correct, and there may be deviations depending on the operator's experience and ability.

### SUMMARY

The present invention is to use an electromagnetic field to accurately and easily understand an inside of a procedure site in an oral cavity and a placement state of an implant, which an operator may not see, in real time. In addition, the operator checks a position and collision of an invisible nerve canal in a patient's oral cavity and is guided to perform the procedure.

In addition, the present invention provides a method and system for guiding of dental implant capable of providing a function of calculating a distance and angle to a section where implant placement is completed and checking in real time whether the implant is accurately placed at a planned placement position, and guiding the implant to be placed at the correct position by informing a risk of a placement depth.

In addition, the present invention provides a method and system for guiding of dental implant capable of controlling a guide program only through motion while holding a handpiece.

In addition, the present invention provides a method and system for guiding of dental implant capable of accurately analyzing a difference between a procedure plan and post-procedure results of dental implant.

The problems of the present invention are not limited to those mentioned above, and other technical problems will be clearly understood by those skilled in the art from the following description.

According to the present invention, a method for guiding of dental implant includes: a first step of visually displaying a CT image on a display so that a position and direction of implant placement according to a procedure plan are identified; a second step of detecting an electromagnetic field from a base by a sensor installed on a handpiece; and a third step of calculating the position and direction of the handpiece with respect to a procedure target area through the electromagnetic field detected by the sensor and visually displaying the position and direction so that an operator checks the position and direction in real time through the display, in which a target to be visually displayed in the third step includes a three-dimensional virtual image of the handpiece that reflects the misaligned position or direction of the handpiece when the position or direction of the handpiece is different from a reference according to the procedure plan.

In the first step, a visual indicator corresponding to the position and direction of the handpiece during the procedure may be displayed at the procedure target site on the CT image.

The visual indicator may be in a form of a target located at an end of a tool mounted on the handpiece, and may be displayed to move together with a change in the position or direction of the handpiece.

The third step may further include displaying a depth display unit including an indicator in proportion to an entry depth of a tool or implant fixture mounted on the handpiece at the procedure target site.

The indicator may be formed in a form of a bar whose length increases in proportion to the entry depth, and the depth display unit may further include a limit depth display indicator at a certain position in a direction in which the indicator extends.

The depth display unit may be vertically arranged on one side of the CT image.

In the third step, an image included in a three-dimensional virtual image of the handpiece may include a body part of the handpiece, a head part formed at an end portion of the body part, and a tool part mounted on the head part, and in the third step, when the handpiece matches the reference according to the procedure plan, a planar appearance of the body part and the head part may be displayed, and when the handpiece does not match the direction according to the procedure plan, side surfaces of the body part, the head part, and the tool part facing the misaligned direction may be displayed.

In the third step, the virtual image corresponding to the tool or fixture mounted on the handpiece may further include displaying each reference image according to the procedure plan according to axial, sagittal, and coronal planes of the procedure target site in the CT image.

When the position or direction of the handpiece is different from the reference according to the procedure plan, for each reference image according to the procedure plan according to the axial, sagittal and coronal planes of the procedure target site, the virtual image corresponding to the tool or fixture mounted on the handpiece may be displayed to have the misaligned position or direction.

When the position or direction of the handpiece is different from the reference according to the procedure plan, for the virtual image corresponding to the tool or fixture mounted on the handpiece, each reference image according to the procedure plan may be displayed to have the misaligned position or direction according to the axial, sagittal, and coronal planes of the procedure target site.

When the position or direction of the handpiece is different from the reference according to the procedure plan, a color of the virtual image corresponding to the tool or fixture mounted on the handpiece may be changed and displayed.

### BRIEF DESCRIPTION OF DRAWINGS

FIG. 1 is a conceptual diagram illustrating a system for guiding a process of real-time implant placement according to an example related to the present invention.
FIG. 2 is a flowchart exemplarily illustrating a procedure process to which a method of guiding a process of real-time implant placement related to the present invention is applied.
FIG. 3 is an example of a screen that may be displayed through a display to guide a progress of real-time implant placement, which is configured to allow a patient's oral CT state to be checked and a procedure plan to be established.
FIG. 4 is an example of a screen that may be output through a display to guide a progress of real-time implant placement, and comprehensively displays several guide screens that may be referenced.
FIG. 5 is an exemplary display screen illustrating a procedure target site displayed on a three-dimensional CT image and a visual indicator, and a depth display unit, respectively.
FIG. 6A is a display screen illustrating an example when a handpiece is placed in a correct position and direction and FIG. 6B is a display screen illustrating an example when the handpiece deviates from a reference.
FIG. 7 is a display screen illustrating an appearance in which the handpiece is manipulated in the state of FIGS. 6A and 6B and returns to a correct position and direction. FIG. 8 is an example related to the present invention, and is screens illustrating an appearance in which a virtual image corresponding to a tool or fixture mounted on a handpiece is displayed for each reference image for each axial, sagittal, and coronal plane in the CT image.
FIG. 9 is screens illustrating an appearance in which a virtual image corresponding to a tool or a fixture mounted on the handpiece when the position or direction of the handpiece deviates from the reference according to a procedure plan in the state of FIG. 8, displayed for each axial, sagittal, and coronal plane.
FIG. 10 is another example related to the present invention, and screens illustrating an appearance in which a virtual image corresponding to a tool or fixture mounted on a handpiece is displayed for each reference image for each axial, sagittal, and coronal plane in the CT image.
FIG. 11 is screens illustrating an appearance in which a virtual image corresponding to a tool or a fixture mounted on the handpiece when the position or direction of the handpiece deviates from the reference according to a procedure plan in the state of FIG. 10, displayed for each axial, sagittal, and coronal plane.
FIGS. 12A and 12B are an embodiment of the present invention, and are conceptual diagrams illustrating an appearance in which an output image is manipulated by a guide program using a handpiece.
FIGS. 13A and 13B are another embodiment of the present invention, and are conceptual diagrams illustrating an appearance in which an output image is manipulated by a guide program using a handpiece.
FIG. 14 is another embodiment according to the present invention, and is a diagram conceptually illustrating a system for guiding of dental implant.
FIG. 15 is a flowchart for describing a method of analyzing an implant placement state through the system of FIG. 14.
FIG. 16 is a perspective view schematically illustrating a sensor according to an example of the present disclosure.
FIGS. 17 and 18 are for describing an arrangement of beads installed in the sensor of FIG. 16., and FIG. 17 is a schematic plan view of the sensor, and FIG. 18 is a schematic front view of the sensor.
FIG. 19 is a diagram schematically illustrating those displayed on a computed tomography image by comparing an appearance before and after implant placement.
FIGS. 20 and 21 are diagrams illustrating an appearance of an implant before and after placement for displaying measurement targets for each analysis type in relation to the present invention.
FIGS. 22A to 22C are diagrams illustrating the appearance of the implant according to a procedure plan and the appearance of the post-procedure implant in the computed tomography image related to the present invention.
FIGS. 23 and 24 are examples related to the present invention, and are screens illustrating before and after placement displayed on the display, respectively.
FIGS. 25A to 25C are screens illustrating individual comparative analysis of implant according to the procedure plan before placement and implant after placement.

### DETAILED DESCRIPTION

Hereinafter, a method and system for guiding a process of real-time implant placement related to the present invention will be described in detail with reference to the attached drawings. Terms used in the specification and claims may not be limited to dictionary meanings, and may be appropriately defined and understood to explain the present invention in the best way.

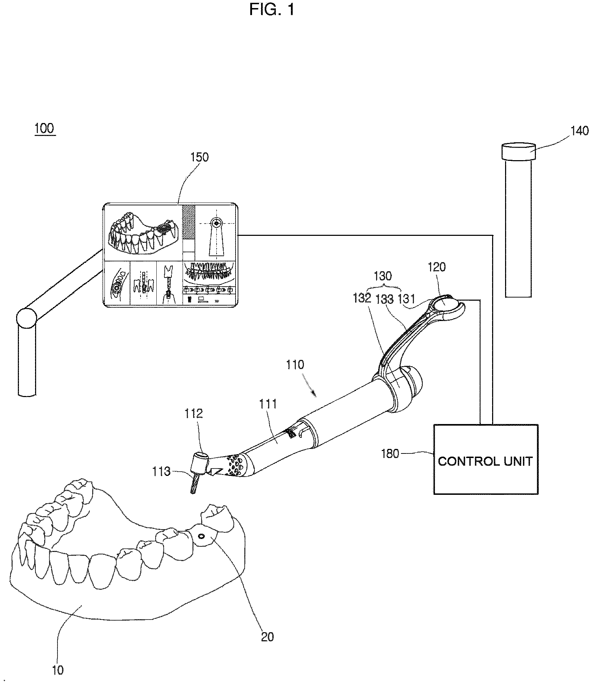

Referring to FIG. 1, a system 100 for guiding a process of real-time implant placement 100 related to the present invention includes a handpiece 110 for performing an implant procedure on a procedure target site 20 of a patient 10, a sensor 120 mounted on the handpiece 110, and a display 150 that may output images for procedure guidance according to a signal from a control unit 180 based on a detection signal from the sensor 120. The sensor 120 detects a magnetic field or an electromagnetic field (EM) generated from a base 140 and transmits a signal to the control unit 180, and the control unit 180 calculates the exact position and direction of the handpiece 110 based on the signal.

The handpiece 110 includes a body part 111 equipped with a driving element therein, a head part 112 formed to allow a tool to be mounted on an end portion of the body part 111, a drill tip 113 mounted on the head part 112, etc. The sensors 120 may be subject to electromagnetic interference due to a driving element inside the body part 111. To minimize the electromagnetic interference, the sensor 120 may be configured to be spatially spaced apart from each other by a spacing jig 130. The spacing jig 130 includes a support part 131 for fixing the sensor 120, a coupling part 132 coupled to the body part 111, and a space extension part 133 connecting between the support part 131 and the coupling part 132. In this case, the spatial extension part 133 separates the sensor 120 from the body part 111 in axial and radial directions, so it is possible to minimize mutual spatial interference with the arrangement of cables connected to the body part 111 while reducing the influence of the electromagnetic interference. The coupling part 132 may be formed in a ring or fork shape so as to be easily coupled to the body part 111 in a state rotated by a selected angle. To reduce the influence of the electromagnetic interference, a non-magnetic material (e.g., titanium, etc.) may be applied to the spacing jig 130 or the handpiece 110 connected to the sensor 120.

The sensor 120 may include a magnetic field sensor. When the sensor 120 detects the magnetic field from the base 140 and transmits the detection result to the control unit 180, the control unit 180 may be configured to understand and track the position and posture of the handpiece 110 through calculation and display the determined and tracked position and posture on the displays 150 or provide necessary feedback to an operator.

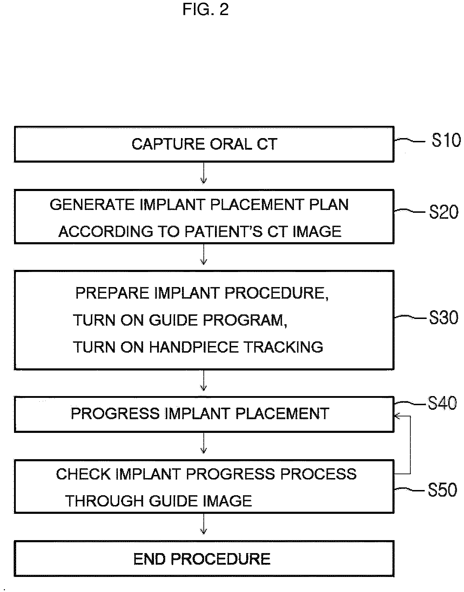

FIG. 2 is a flowchart exemplarily illustrating a procedure process to which a method of guiding a process of real-time implant placement related to the present invention is applied. CT scan information and real-time motion tracking information of the handpiece are used at each procedure stage through a guide program and displayed on the display, allowing an operator to refer to and confirm them.

First, a CT scan is performed to obtain information on a patient's oral condition (S10), and an operator generates an implant placement plan through a guide program (S20) . When the procedure begins, the guide program is activated and the handpiece tracking function is executed (S30). The operator performs placement of an implant using a handpiece that is motion-tracked by a sensor that detects an electromagnetic field (S40) . The position and direction of the handpiece, which is tracked in real time, is output through the display in comparison with the procedure plan based on the CT scan image, and the operator checks the placement progress process through the guide image (S50) . By repeating this process, the procedure is completed when the placement is achieved at the depth and direction planned for the procedure.

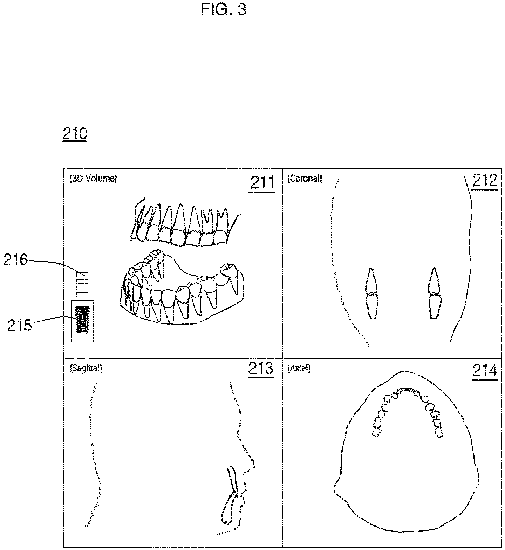

FIG. 3 is an example of a screen 210 that may be displayed through a display to guide a progress of real-time implant placement, which is configured to allow a patient's oral CT state to be checked and a procedure plan to be established. That is, a captured CT image 211, a coronal image 212, a sagittal image 213, and an axial image 214 output around the procedure site may be arranged within the entire screen 210. A display window, widget, or the like for checking patient information may be provided on one side of the screen 210. The guide program may include a screen control user interface for the operator to obtain appropriate image information of the 3D CT image 211 and the 2D images 212, 213, and 214. Based on these patient images, the operator establishes an implant placement plan (selection of fixture type, selection of placement position, virtual placement considering neural canal 30, etc.). The fixture 215 and the related information list 216 may also be configured to be displayed on the screen 211.

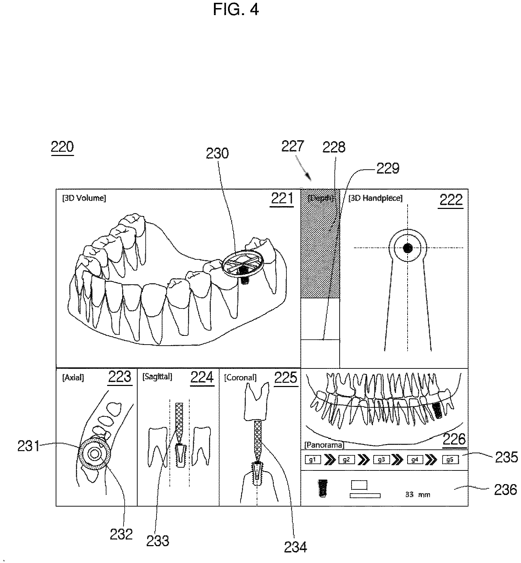

FIG. 4 is an example of a screen 220 that may be output through a display to guide a real-time implant placement progress, in which several guide screens 221, 222, 223, 224, 225, 226, 227, 235, and 236 are comprehensively displayed. These guide screens may be displayed in whole or in part as needed, or the composition, order, or the like of arrangement may be selected, changed, or adjusted.

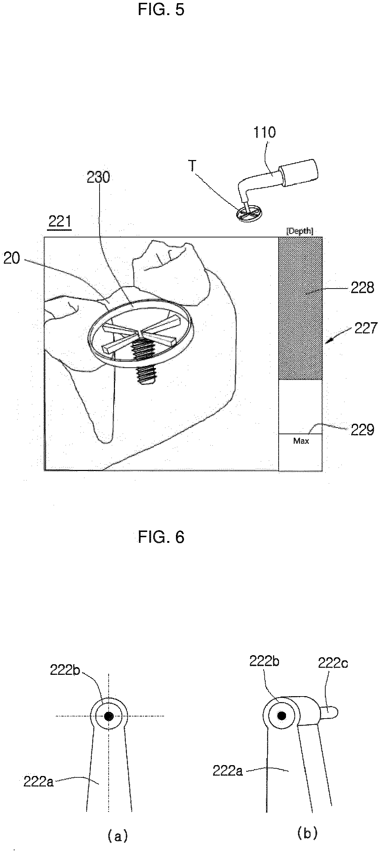

The first screen 221 shows a visual indicator 230 corresponding to the position and direction of the handpiece 110 during the procedure displayed on the CT image of the procedure target site 20. The visual indicator 230 is in the form of a target T located at an end of the tool mounted on the handpiece 110 as illustrated in FIG. 5, and is displayed to move together with changes in the position or direction of the handpiece 110. This visual indicator 230 may reduce the possibility of the procedure target site 20 being covered when the shape of the handpiece 110 is expressed in the procedure target site 20 as it is, and can express not only the position but also the direction.

A depth display unit 227 may be arranged on one side of the first screen 211. This depth display unit 227 includes an indicator 228 that is proportional to an entry depth of the procedure target site 20 of the tool or fixture mounted on the handpiece 110. The indicator 228 is in the form of a bar whose length increases in proportion to the entry depth. As the depth increases, the indicator 228 also extends from top to bottom. To easily check this, the depth display unit 227 may be arranged vertically on one side of the CT image. An operator may intuitively understand the entry depth through the bar-shaped indicator 228. The depth display unit 227 may include a limit depth display indicator 229 at a certain position in the direction in which the indicator 228 extends. The limit depth display indicator 229 corresponds to the position of the neural canal 30 and functions as a visual stopper to prevent the operator from proceeding with drilling further than that. The indicator 228 may be configured to provide a warning to the operator about the procedure depth by changing color or displaying it in a blinking form (e. g., changing from green to red and blinking) when entering the reference depth or more. Such warnings may utilize auditory means as well as visual means.

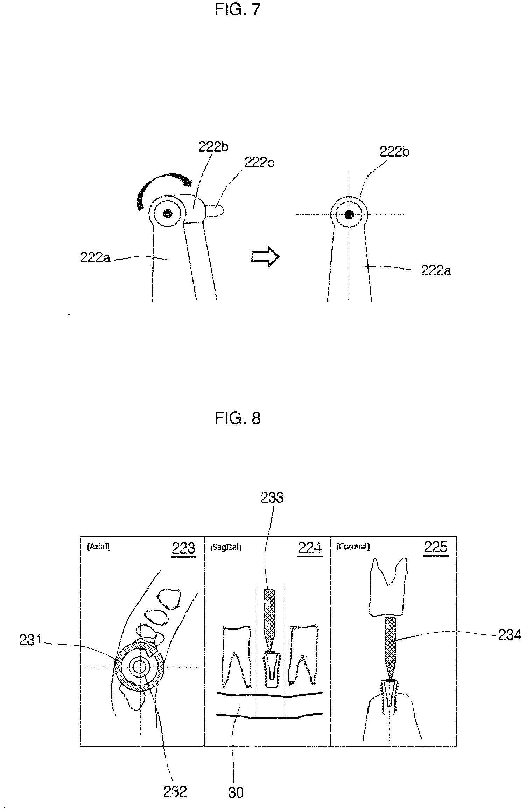

The second screen 222 is configured to immediately check whether the position and direction of the handpiece 110 are visually aligned with the position and direction that matches the procedure plan. To this end, a three-dimensional virtual image of the handpiece 110 reflecting the misaligned position or direction of the handpiece 110 is output on the second screen 222. As illustrated FIGS. 6A and 6B, an image included in the three-dimensional virtual image of the handpiece appears similar to the actual shape of the handpiece 110 by including a body part 222a, a head part 222b, and a tool part 222c. As illustrated in FIG. 6A, when the handpiece is arranged in the correct position and direction, plane views of the body part 222a and head part 222b are displayed, but as illustrated in FIG. 6B, when the handpiece does not match the direction according to the procedure plan, side surfaces of the body part 222a, the head part 222b, and the tool part 222c facing the misaligned direction are also displayed. In this case, the operator may immediately identify the wrong direction (intuitively) just as it would in reality, only by looking at the image, and may correct and modify and return the identified wrong direction to the correct position by immediately rotating the handpiece in the opposite direction, as illustrated in FIG. 7.

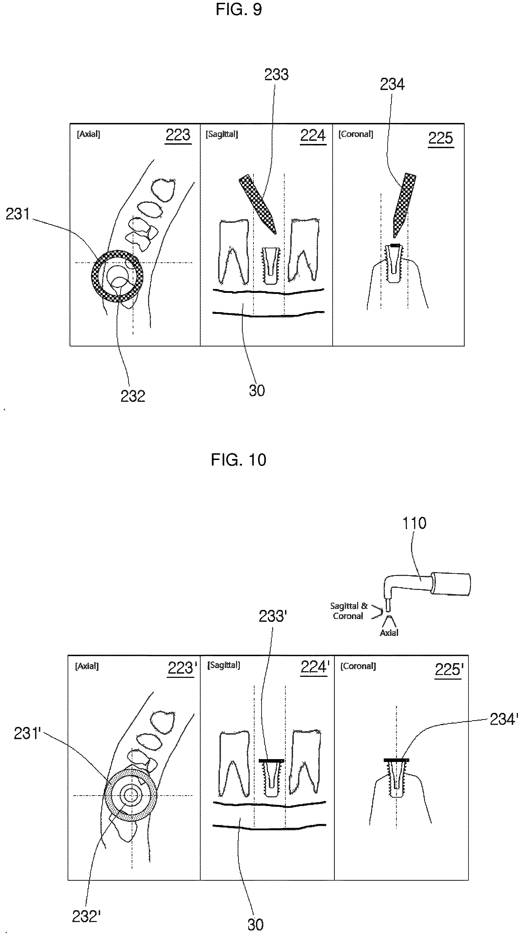

In the third screen 223, the fourth screen 224, and the fifth screen 225, virtual images 232, 233, and 234 corresponding to tools or fixtures mounted on the handpiece are displayed for each reference image according to the procedure plan by axial, sagittal, and coronal planes of the procedure target site in the CT image. Like the third screen 223, the axial image may be configured to display a virtual image 232 and a separate indicator 231 that may easily identify the position with color. FIG. 8 illustrates a case where the position and direction of the handpiece matches the reference according to the procedure plan, and FIG. 9 illustrates a case where the position or direction of the handpiece deviates from the reference according to the procedure plan. As illustrated in FIG. 9, the virtual images 232, 233, and 234 corresponding to the tools or fixtures mounted on the handpiece are displayed to have a misaligned direction with respect to the reference image. Even in this case, the virtual images 232, 233, and 234 may be configured to change from green to red and blink to provide a warning to the operator.

The third screen 223, fourth screen 224, and fifth screen 225 of FIG. 9 may be configured to display a degree and distance between the fixture and the handpiece to allow an operator to understand a degree of deviation.

Referring back to FIG. 4, one side of the entire screen 220 may include a sixth screen 226 that provides a panoramic view containing the entire tooth. Various guide screens output through the display may be different in the operator's preferred method, and thus, may be autonomously selected and used by an operator. The guide program may have a user interface that allows a user to change a screen arrangement by selection.

A seventh screen 235 that guides the tool to be selected according to the drilling sequence may be displayed on one side of the entire screen 220, and an eighth screen 236 that displays information related to the fixture being used may also be output.

Unlike FIGS. 8 and 9, FIGS. 10 and 11 illustrate that, when the position or direction of the handpiece 110 does not match the reference according to the procedure plan, each reference image according to the procedure plan is displayed to have a position or direction that deviates from virtual images 232', 233', and 234 ' corresponding to the tool or fixture mounted on the handpiece. That is, in this example, it is assumed that there is a camera arranged in a mutually orthogonal direction at an end of the drill, and the real-time procedure situation in the relevant area from the perspective of the drill is visually implemented.

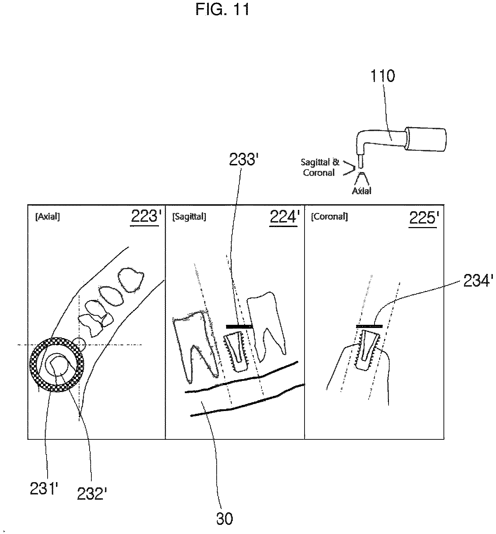

Even in FIG. 11, the third screen 223' , the fourth screen 224', and the fifth screen 225' may be configured to display a degree and distance between the fixture and the handpiece to allow an operator to understand a degree of deviation.

The method and system for guiding a process of real-time implant placement and system described as described above is not limited to the configuration and method of the described embodiments. All or some of the respective exemplary embodiments may be selectively combined with each other so that the above-mentioned exemplary embodiments may be variously modified to substitutable equivalents.

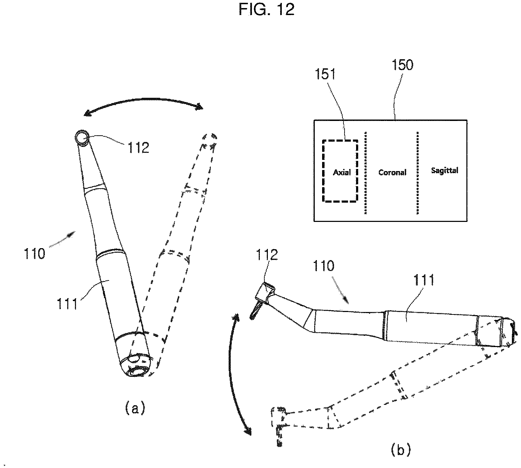

FIGS. 12A and 12B are another embodiment according to the present invention, and illustrate an appearance in which the handpiece 110 is used to manipulate the image output on the display 150 by a guide program.

A method for guiding of dental implant in this example includes displaying a plurality of display windows or sequences related to the implant procedure on the display 150, detecting the movement of the handpiece 110 according to the detection result of the sensor 120, and when the detected movement of the handpiece 110 matches a pre-stored motion, changing from a previously selected display window or sequence in response to the movement and displaying the selected result on the display 150.

FIGS. 12A and 12B illustrate an interface method of the plurality of display windows, in which an axial image, a coronal image, and a sagittal image displayed horizontally are displayed on the display 150. The currently selected image is the axial image indicated by the indicator 151. In this state, when a user holds the handpiece 110 and makes a motion (yaw) to swing the head part 112 to the left and right in the forward and reverse directions above a reference angle around the body part 111, an indicator 151 for selection moves in that direction (FIG. 12A) . If the indicator 151 moves to the selected image, then a motion (pitch) is made to rotate the head part 112 up and down above the reference angle around the body part 111 as illustrated in FIG. 12B, and therefore, details of the selected image are displayed are displayed.

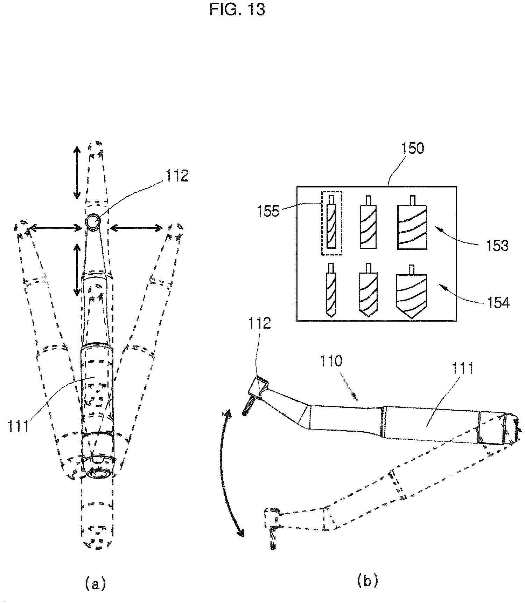

FIGS. 13A and 13B illustrate a screen interface method in the case where the procedure is performed according to a plurality of sequences during the implant placement process, and drills 153 and 154 arranged by diameter are displayed. In order to select the desired drill, an operator may change the position of the indicator 155 by holding the handpiece 110 and moving the handpiece 110 back and forth and left and right above a reference distance in the horizontal direction (FIG. 13A). When the drill is selected by moving the indicator 155, as with FIGS 12A and 12B, the user can display details by rotating (yaw) the head part 112 up and down above the reference angle around the body part 111 (FIG. 12B).

When the procedure is performed using this method, there is no need for the operator to remove medical gloves or mount the handpiece to control a PC, so but also safety of the procedure is guaranteed, and the procedure is hygienically safe. In addition, it may be used for educational purposes by doctors and dental hygienists participating in implant procedure.

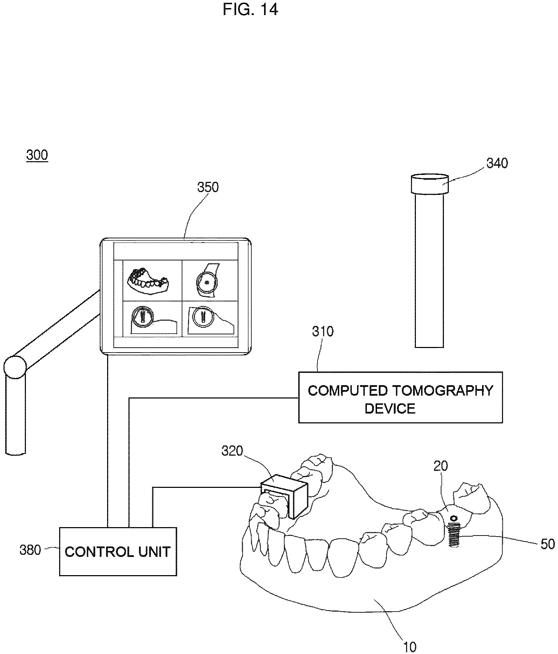

FIG. 14 is another embodiment according to the present invention, and is a diagram conceptually illustrating a system for guiding of dental implant.

Referring to FIG. 14, a system 300 for guiding of dental implant in this example includes elements for performing and analyzing an implant procedure on the procedure target site 20 of a patient 10. A computed tomography device 310 for capturing the appearance of the patient 10 including the procedure target site 20 before and after placing the implant 50 (also called 'fixture') in the procedure target site 20 of the patient 10 is provided.

The control unit 380 is configured to receive images captured by the computed tomography device 310 and output the images through the display 350, or to perform an analysis process before and after implant placement based on the CT images. The control unit 380 may refer to a functional module related to analysis before and after implant placement in an implant placement guide software or program.

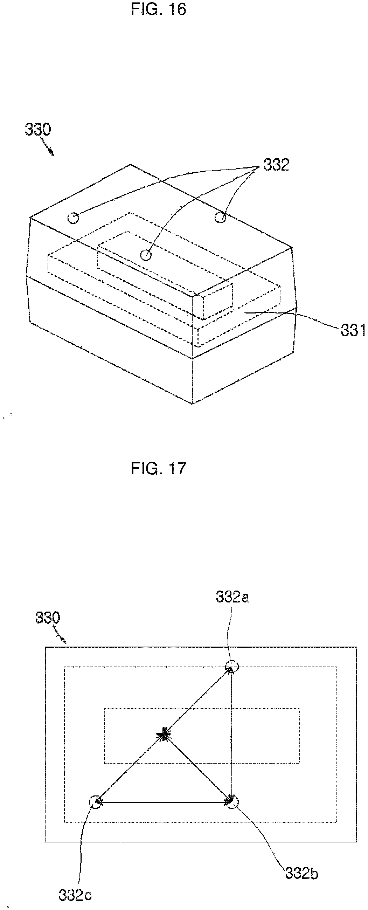

In this embodiment, a jig 320 is configured to be fixed around the procedure target site 20 so that it can be analyzed whether the implant 50 has been accurately placed in the procedure target site 20 by comparing with the procedure plan. The jig 320 may include a sensor 330 (see FIG. 16), which will be described later. The sensor 330 included in the jig 320 is configured to detect a magnetic field or an electromagnetic field (EM) generated from the base 340. The jig 320 tracks changes in the position or direction of the procedure target site 20 according to changes in the movement or posture of the patient 10 to provide accurate information on the procedure situation to the operator or when operating a guide robot, etc. , may guide the accurate movement or path of a robot when operating a guide robot, etc. The jig 320 may include a bracket-like shape, an adhesive element, or the like for fixing to a body part such as a tooth, or may be configured so that the sensor 330 is directly attached to the tooth. The jig 320 may be called a 'tooth bite' in some cases.

The display 350 is configured to output images captured by the computed tomography device 310 and various information related to the procedure, or to visually display comparative analysis results before and after implant placement according to a signal from the control unit 380.

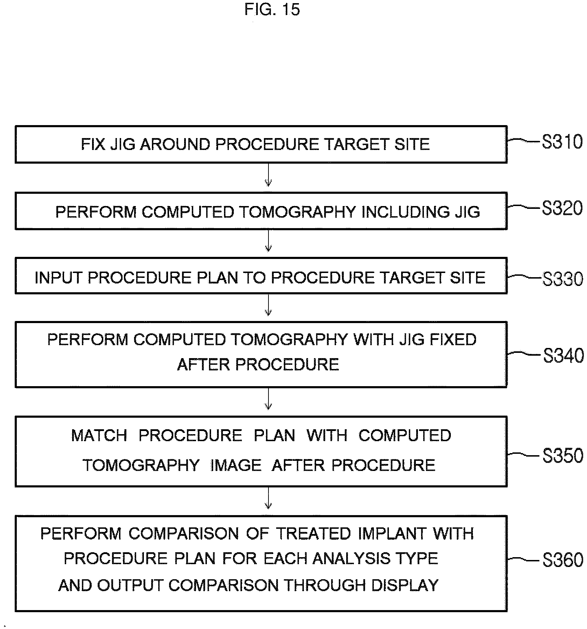

The implant placement analysis method using the system illustrated in FIG. 14 will be described with reference to FIG. 15 and below.

To analyze whether the implant has been accurately placed, first, the jig 320 described above is fixed around the procedure target site 20 (S310) . The jig 320 may be provided with the sensor 330 described above, thereby detecting the changes in the position or direction of the procedure target site 20.

After fixing the jig 320 to the teeth of the patient 10 or the like, a CT scan is performed on the jig 320 along with the procedure target site 20 using the computed tomography device 310 (S320). As illustrated in FIGS. 16 to 18, the jig 320 may detect the real-time position or direction of the procedure target site 20 to be detected by the sensor 330 included therein. In this case, the jig 320 is provided with a plurality of beads 332, 332a, 332b, and 332c that may provide an image that can be distinguished from a living body during computed tomography. The beads 332, 332a, 332b, and 332c may be formed in the form of metal beads having magnetism. Accordingly, in the case of the computed tomography, the positions and forms of the beads 332, 332a, 332b, and 332c may be identified by color (e.g., white) that is distinguished from a living body (light or dark gray) in images ranging from black to white expressed by hounsfield scale (HU) values. In this case, as illustrated in FIG. 16, the three beads 332a, 332b, and 332c may be placed equidistantly in a triangular shape when viewed in a plan view with respect to the magnetic sensor 333 attached to the bottom of the circuit board 331, and may be arranged to achieve a certain height difference with respect to the magnetic sensor 333. In order to reduce the magnetic interference, the three beads 332a, 332b, and 332c may be arranged on the surface of the housing or case that protects the sensor 330. Since a plane and coordinate axis are generated by these beads 332, 332a, 332b, and 332c, the position of the sensor may be detected or registration may be performed by comparing the coordinate axis before and after the procedure.

After obtaining the first image including the jig 320 by the computed tomography, the operator makes a plan including the position (including depth and horizontal position) and direction of the implant to be placed and inputs the procedure plan through the user interface (S330). The control unit 380 may be configured to store the procedure plan and output the stored procedure plan through the display 350.

According to the procedure plan, the operator performs the implant placement procedure. In this case, during the procedure, the sensor 330 included in the jig 320 may be configured to provide detection information so that the exact position or direction relationship between the procedure target site 20 and the handpiece held by the operator may be understood in real time.

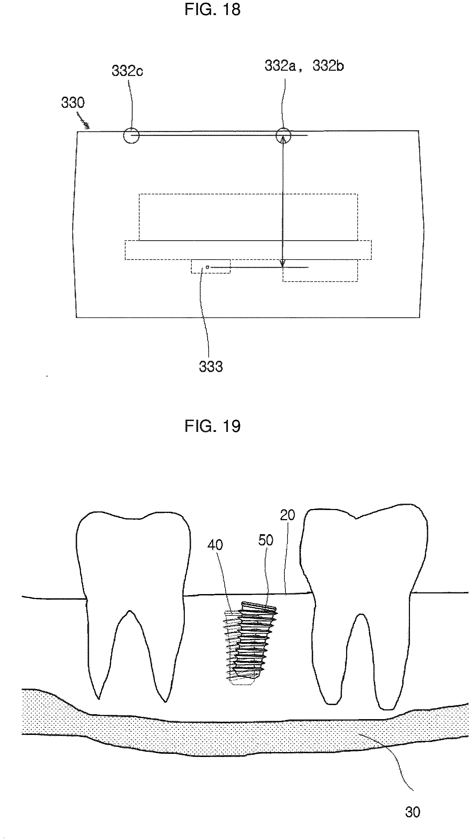

After the procedure is completed, capturing is performed with the jig 320 fixed using the computed tomography device 310 to analyze the placed implant (S340). The second image thus obtained also includes the beads 332a, 332b, and 332c, and the coordinate axis and plane of the beads 332a, 332b, and 332c in the second image are registered with the coordinate axis and plane of the beads 332a, 332b, and 332c in the first image (S350). As a result, the comparative analysis between the implant 40 according to the procedure plan (see FIG. 19) and the post-procedure implant 50 is possible.

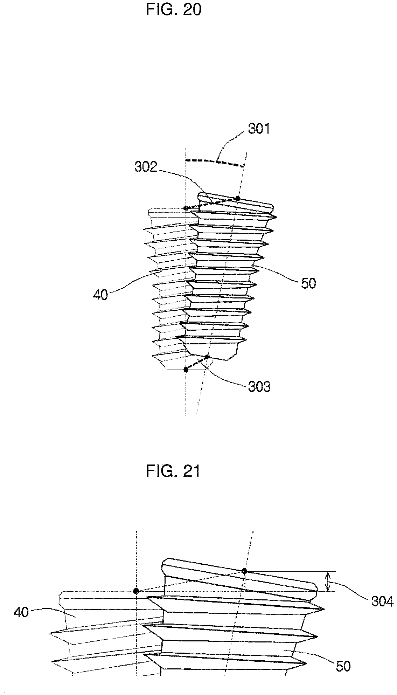

Next, the comparison between the implant 40 according to the procedure plan included in the second image and the post-procedure implant 50 is performed for each type and output through the display 350 (S360). FIGS. 20 and 21 are diagrams illustrating an appearance of an implant before and after placement for displaying measurement targets for each analysis type in relation to the present invention. Both the implant 40 in the procedure plan and the implant 50 after placement have a three-dimensional position and direction by the computed tomography, and the accurate value may be obtained from the reference point using the jig 320. The analysis types of the implant placement results may include the following four types.(1) Angular Deviation

A three-dimensional angle 301 of the central axis of the procedure planned implant 40 and the central axis of the implant 50 after placement may be measured, and the analysis results may be displayed or stored on the screen to check the difference from the procedure plan.

### (2) Signed Depth

A vertical distance 304 of the upper end of the central axis of the procedure planned implant 40 and the upper end of the central axis of the implant 50 after placement may be measured, and the analysis results may be displayed or stored on the screen to check the difference from the procedure plan. This distance 304 may be reference information on how deeper or shallower the implant is placed than the procedure plan.

### (3) Coronal Deviation

A three-dimensional distance 302 of the upper end of the central axis of the procedure planned implant 40 and the upper end of the central axis of the implant 50 after placement may be measured, and the analysis results may be displayed or stored on the screen to check the difference from the procedure plan.

### (4) Apical Deviation

A three-dimensional distance 303 of a lower end of the central axis of the procedure planned implant 40 and a lower end of the central axis of the implant 50 after placement may be measured, and the analysis results may be displayed or stored on the screen to check the difference from the procedure plan.

In this way, it is possible to check the state of the implant procedure in detail and accurately through the provided comparison between the implant 40 in the procedure plan and the implant 50 after placement for each type of analysis.

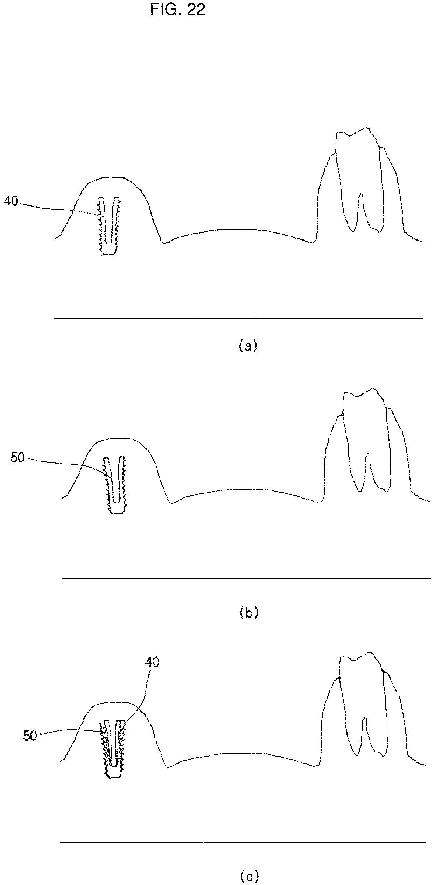

FIGS. 22A to 22C are diagrams illustrating the appearance of the implant according to a procedure plan and the appearance of the post-procedure implant in the computed tomography image related to the present invention. In this example, FIGS. 22A and 22B illustrate each arbitrary cross section that allow relative comparison between the implant 40 according to the procedure plan and the actually placed implant 50, respectively, or FIG. 22C illustrates that the difference may be identified by overlapping the implant 40 according to the procedure plan and the post-procedure implant 50.

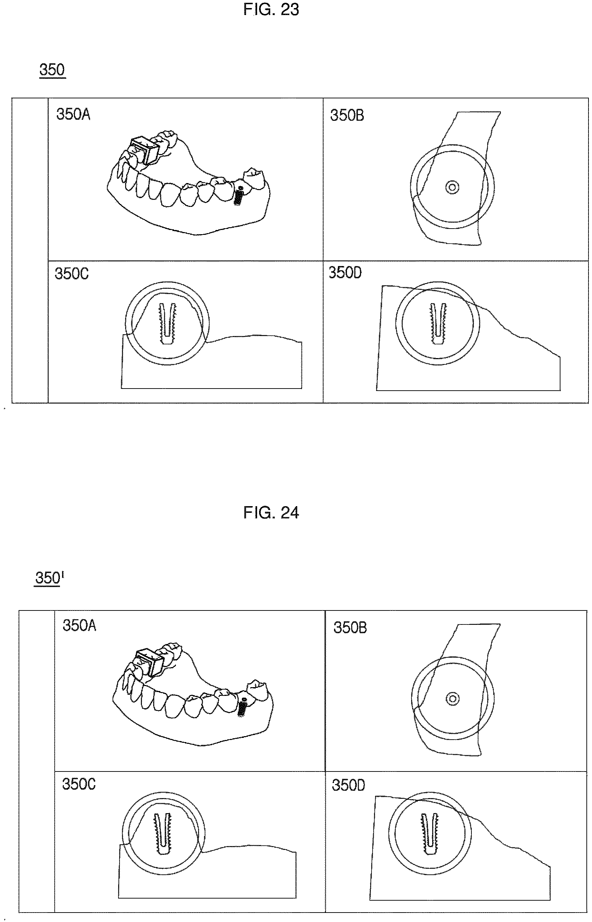

FIGS. 23 and 24 are examples related to the present invention, and are screens illustrating before and after placement displayed on the display, respectively.

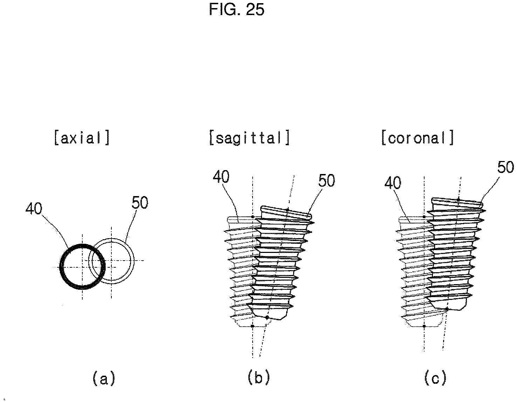

In this example, the screens 350 and 350' are configured so that the plan before the procedure and the state of the post-procedure implant can be individually confirmed through several split screens 350A, 350B, 350C, and 350D. The comparison of each split screen included in these screens 350 and 350' will be described with reference to FIGS. 25A to 25C.

FIGS. 25A to 25C are screens illustrating individual comparative analysis of implant according to the procedure plan before placement and implant after placement.

In this example, from the viewpoint of the axial, sagittal, and coronal planes within the screen all at once or individually, it is shown that the misalignment in position or direction between the implant 40 according to the procedure plan and the implant 50 after placement may be accurately presented through the distance or angle.

In this way, the computed tomography is performed before and after placement with the jig 320 fixed, and the image registration is performed to compare the procedure planned implant 40 and the post-procedure implant 50 to obtain accurate measurement and analysis results for each type of analysis. When the implant placement training or procedure testing is performed, the objective and accurate check is possible during evaluation and verification before and after placement.

According to a method and system for guiding of dental implant related to the present invention, by tracking the exact position and direction of a handpiece in real time using an electromagnetic field sensor attached to the handpiece, it is possible for an operator to perform a procedure while checking a neural canal in real time not only at the beginning of implant placement but also while the implant placement is in progress. By applying this technology, when performing dental implant placement surgery, the operator can check the progress of the placement while checking the 2D and 3D CT images in real time. The placement process can be checked on CT images and the implant can be placed while taking into account the surrounding nerve canal.

According to an example related to the present invention, when the position or direction of the handpiece is different from the reference according to the procedure plan, by outputting a three-dimensional virtual image of a handpiece reflecting the misaligned position or direction of the handpiece, it is possible for an operator to intuitively identify the misaligned position or direction during the procedure.

According to an example related to the present invention, by displaying a depth display unit including an indicator in proportion to an entry depth of a tool or implant fixture mounted on a handpiece in a procedure target site on a display, it is possible for an operator to immediately check the depth without confusion and safely perform a procedure without touching a neural canal.

According to another example related to the present invention, when the detected movement of the handpiece matches the pre-stored motion, it is configured to display the selected result on the display by changing from the previously selected display window or sequence in response to the movement, so, when a procedure is performed, there is no need for an operator to remove medical gloves or mount a handpiece to control the PC, so not only convenience but also safety of the procedure is guaranteed, and the procedure is hygienically safe. In addition, a screen interface method and system for implant placement related to the present invention has the advantage of being able to be used for educational purposes by a doctor, a dental hygienist, or the like participating in an implant procedure.

According to another method and system for guiding of dental implant related to the present invention, computed tomography is performed before and after placement, respectively, with a jig fixed and image registration is performed, so it is possible to obtain accurate measurement and analysis results for each type of analysis when comparing the post-procedure implant with the procedure plan. Using this method, when placement training or procedure testing of the implant is in progress, objective and accurate confirmation is possible at the time of evaluating and verifying before and after placement. In addition, it is possible to improve the reliability of the procedure by sharing the placement state or results with a patient.

A method and system for guiding of dental implant described above is not limited to the configuration and method of the described embodiments. All or some of the respective exemplary embodiments may be selectively combined with each other so that the above-mentioned exemplary embodiments may be variously modified to substitutable equivalents.

## Claims

1. A method for guiding of dental implant, comprising:
a first step of visually displaying a CT image on a display so that a position and direction of implant placement according to a procedure plan are identified;
a second step of detecting an electromagnetic field from a base by a sensor installed on a handpiece; and
a third step of calculating the position and direction of the handpiece with respect to a procedure target site through the electromagnetic field detected by the sensor and visually displaying the position and direction so that an operator checks the position and direction in real time through the display,
wherein a target to be visually displayed in the third step includes a three-dimensional virtual image of the handpiece that reflects the misaligned position or direction of the handpiece when the position or direction of the handpiece is different from a reference according to the procedure plan.

2. The method of claim 1, wherein, in the first step, a visual indicator corresponding to the position and direction of the handpiece during the procedure is displayed at the procedure target site on the CT image.

3. The method of claim 2, wherein the visual indicator is in a form of a target located at an end of a tool mounted on the handpiece, and is displayed to move together with a change in the position or direction of the handpiece.

4. The method of claim 1, wherein the third step further includes displaying a depth display unit including an indicator in proportion to an entry depth of a tool or implant fixture mounted on the handpiece at the procedure target site.

5. The method of claim 4, wherein the indicator is formed in a form of a bar whose length increases in proportion to the entry depth, and the depth display unit further includes a limit depth display indicator at a certain position in a direction in which the indicator extends.

6. The method of claim 4, wherein the depth display unit is vertically arranged on one side of the CT image.

7. The method of claim 1, wherein, in the third step, an image included in a three-dimensional virtual image of the handpiece includes a body part of the handpiece, a head part formed at an end portion of the body part, and a tool part mounted on the head part, and
in the third step, when the handpiece matches the reference according to the procedure plan, a planar appearance of the body part and the head part is displayed, and when the handpiece does not match the direction according to the procedure plan, side surfaces of the body part and the head part facing the misaligned direction are displayed.

8. The method of claim 1, wherein in the third step, the virtual image corresponding to the tool or fixture mounted on the handpiece further includes displaying each reference image according to the procedure plan according to axial, sagittal, and coronal planes of the procedure target site in the CT image.

9. The method of claim 8, wherein, when the position or direction of the handpiece is different from the reference according to the procedure plan, for each reference image according to the procedure plan according to the axial, sagittal and coronal planes of the procedure target site, the virtual image corresponding to the tool or fixture mounted on the handpiece is displayed to have the misaligned position or direction.

10. The method of claim 8, wherein, when the position or direction of the handpiece is different from the reference according to the procedure plan, for the virtual image corresponding to the tool or fixture mounted on the handpiece, each reference image according to the procedure plan is displayed to have the misaligned position or direction according to the axial, sagittal, and coronal planes of the procedure target site.

11. The method of claim 1, wherein, when the position or direction of the handpiece is different from the reference according to the procedure plan, a color of the virtual image corresponding to the tool or fixture mounted on the handpiece is changed and displayed.

12. A method for guiding of dental implant, comprising:
displaying a plurality of display windows or sequences related to an implant procedure on a display, at least one of the plurality of display windows or sequences being selected by changing from a previously selected display window or sequence;
detecting movement of the handpiece according to a detection result of a sensor installed on the handpiece; and
when the sensed movement of the handpiece matches a pre-stored motion, changing from a previously selected display window or sequence in response to the movement and displaying the selected result on the display.

13. The method of claim 12, wherein the sensor is configured to detect an electromagnetic field from a base that generates the electromagnetic field.

14. The method of claim 12, wherein the plurality of display windows or sequences include an axial image, a coronal image, and a sagittal image.

15. The method of claim 14, wherein the handpiece includes a body part extending in a longitudinal direction and a head part formed to mount a tool on an end portion of the body part, and
the pre-stored motion includes a motion (yaw) that swings the head part left and right in forward and reverse directions around the body part by more than a reference angle, and a motion (pitch) that rotates the head part up and down by more than a reference angle around the body part.

16. The method of claim 12, wherein the plurality of display windows or sequences include a plurality of drills arranged by diameter according to an order of the procedure.

17. The method of claim 16, wherein the handpiece includes a body part extending in a longitudinal direction and a head part formed to mount a tool on an end portion of the body part, and
the pre-stored motion includes a motion that moves the head part horizontally back and forth and left and right, respectively, and a motion that rotates the head part up and down by more than a reference angle around the body part.

18. A method for guiding of dental implant, comprising:
obtaining a first image obtained by computed tomography while fixing a jig around a procedure target site;
receiving a procedure plan including a position and direction of an implant to be placed using the first image;
obtaining a second image obtained by computed tomography with the jig fixed after the procedure;
registering the procedure plan with the second image based on the jig; and
comparing a post-procedure implant included in the second image with the procedure plan for each type of analysis.

19. The method of claim 18, wherein the jig includes a sensor configured to detect an electromagnetic field from a base that generates the electromagnetic field.

20. The method of claim 19, wherein the sensor includes a plurality of metal beads with magnetism that provides an image that is distinguished from a living body by the computed tomography.

21. The method of claim 18, wherein the registering of the procedure plan with the second image based on the jig includes the procedure plan includes displaying the procedure plan by applying a relative position and direction of the procedure plan to a plane and a coordinate axis formed by the plurality of beads in the first image to a plane and a coordinate axis formed by the plurality of beads in the second image.

22. The method of claim 18, wherein the comparing of the post-procedure implant included in the second image with the procedure plan for each analysis type includes measuring a three-dimensional angle of a central axis of the implant in the procedure plan and a central axis of the implant after placement.

23. The method of claim 18, wherein the comparing of the post-procedure implant included in the second image with the procedure plan for each analysis type includes measuring and displaying a vertical distance between an upper end of the central axis of the implant in the procedure plan and an upper end of the central axis of the implant after placement.

24. The method of claim 18, wherein the comparing of the post-procedure implant included in the second image with the procedure plan for each analysis type includes measuring and displaying a three-dimensional distance between an upper end of the central axis of the implant in the procedure plan and an upper end of the central axis of the implant after placement.

25. The method of claim 18, wherein the comparing of the post-procedure implant included in the second image with the procedure plan for each analysis type includes measuring and displaying a three-dimensional distance between a lower end of the central axis of the implant in the procedure plan and a lower end of the central axis of the implant after placement.

26. The method of claim 18, wherein the comparing of the post-procedure implant included in the second image with the procedure plan for each analysis type includes distinguishing and displaying the position and direction of the post-procedure implant and the implant of the procedure plan according to an axial image, a sagittal image, and a coronal image.

27. A computer-readable storage medium storing a program configured to execute the method for guiding of dental implant of any one of claims 1 to 26.

28. A system for guiding of dental implant, comprising:
a handpiece provided with a sensor detecting an electromagnetic field from a base;
a display configured to output a CT image so that a position and direction of implant placement according to a procedure plan are identified; and
a control unit configured to calculate a position and direction of the handpiece with respect to a procedure target site through the electromagnetic field detected by the sensor and visually display the position and direction through the display so that an operator checks the position and direction in real time,
wherein, when the position or direction of the handpiece is different from a reference by the procedure plan, the control unit controls the display to display a three-dimensional virtual image of the handpiece that reflects the misaligned position or direction of the handpiece.

29. A system for guiding of dental implant, comprising:
a display configured to display a plurality of display windows or sequences related to an implant procedure on a display, at least one of the plurality of display windows or sequences being selected by changing from a previously selected display window or sequence;
a handpiece provided with a sensor detecting an electromagnetic field from a base; and
a control unit configured to track the handpiece by calculating a position and direction of the handpiece with respect to a procedure target site through an electromagnetic field detected by the sensor,
wherein, when the sensed movement of the handpiece matches a pre-stored motion, the control unit controls the display to display the selected result by changing from a previously selected display window or sequence in response to the movement.

30. A system for guiding of dental implant, comprising:
a computed tomography device configured to capture a procedure target site before and after procedure;
a jig configured to be fixed around a procedure target site and captured together by the computed tomography device, and include a plurality of beads providing an image distinguished from a living body;
a display configured to display an image related to an implant procedure; and
a control unit configured to register a procedure plan with a second image with respect to the jig from a first image obtained by computed tomography with the jig fixed around the procedure target site, the procedure plan including the position and direction of the implant to be placed in the first image input through the user interface, and the second image obtained by computed tomography with the jig fixed after the procedure, and provide an analysis result by comparing the post-procedure implant included in the second image with the procedure plan for each analysis type.
